(19)

Europäisches
Patentamt
European
Patent Office
Office européen
des brevets

(11) **EP 1 916 230 B1**

(12) **EUROPEAN PATENT SPECIFICATION**

(45) Date of publication and mention
of the grant of the patent:
**09.12.2009 Bulletin 2009/50**

(51) Int Cl.:
*C07C 5/333* (2006.01)     *C07C 5/48* (2006.01)
*C07C 11/06* (2006.01)

(21) Application number: **07253961.2**

(22) Date of filing: **05.10.2007**

(54) **Integrated catalytic process for converting alkanes to alkenes and catalysts useful for same**

Integriertes katalytisches Verfahren zur Umwandlung von Alkanen in Alkene und Katalysatoren dafür

Procédé catalytique intégré pour la conversion d'alcanes en alcènes et catalyseurs utiles pour ledit procédé

(84) Designated Contracting States:
**DE FR GB IT**

(30) Priority: **16.10.2006 US 851960 P**

(43) Date of publication of application:
**30.04.2008 Bulletin 2008/18**

(73) Proprietor: **Rohm and Haas Company**
**Philadelphia, PA 19106-2399 (US)**

(72) Inventors:
• **Benderly, Abraham**
**Elkins Park**
**Pennsylvania 19027 (US)**

• **Ruettinger, Wolfgang**
**East Windsor**
**New Jersey 08520 (US)**

(74) Representative: **Buckley, Guy Julian et al**
**Rohm and Haas Europe Services ApS - UK**
**Branch**
**European Patent Department**
**4th Floor**
**22 Tudor Street**
**London EC4Y 0AY (GB)**

(56) References cited:
**DE-A1- 3 439 176     US-A- 3 904 703**
**US-A- 5 439 859**

**Description**

Field of the Invention

The present invention relates to processes for catalytic conversion of alkanes to their corresponding alkenes, as well as catalysts suitable for use in such processes.

Background of the Invention

Well-known commercial processes for the production of monomers, such as unsaturated carboxylic acids and unsaturated nitriles, typically start with one or more alkenes and convert them, by catalytic vapor phase oxidation, to the desired monomer products. In view of the pressures exerted by competition in the industry, and the price difference between alkanes and their corresponding alkenes, such as propane and propene, respectively, efforts are being made to develop processes in which an alkane is used as the starting material to, ultimately, produce the desired monomers at a lower overall cost.

One process modification, which has enjoyed some success in commercial industry, is to simply add an upstream reaction stage in which an alkane is first converted to the corresponding alkene, in the presence of a suitable catalyst. The resulting alkene (e.g., propene) is then fed to the customary oxidation reaction stages, for oxidation of the alkene (e.g., first to acrolein and then to the desired monomer product, as in the two-step oxidation of propene to form acrylic acid). For example, both European Patent Application No. EP0117146 and US Patent No. 5,705,684 describe multi-stage catalytic processes for converting an alkane (propane) to the corresponding unsaturated carboxylic acid (acrylic acid) which includes an initial alkane-to-alkene conversion stage having one or more suitable catalysts to produces a product stream comprising alkene, which is fed to one or more downstream oxidation stages. Various catalysts and methods are known to catalyze conversion of alkanes to their corresponding alkenes.

There are catalysts known which catalyze oxidative dehydrogenation of an alkane, which is an exothermic reaction and, by definition, occurs in the presence of oxygen. For example, US Patent No. 5,086,032 describes oxidative dehydrogenation of propane over nickel-molybdenum-based oxide catalysts. Another category of known oxidative dehydrogenation catalysts is disclosed in U.S. Patent Application Publication No. 2002/0077518, which are manganese-based multi-metal oxides with one or more oxides of antimony, tungsten and chromium, as well as optional additional oxides including, but not limited to, oxides of nickel, cobalt, niobium, tantalum, cesium, lithium, sodium and potassium.

As described in U.S. Patent Application Publication No. 2004/0034266 ("US '266"), catalytic oxidative dehydrogenation of propane and ethane has been performed in microchannel reactors where the microchannels contain a magnesium-vanadium-based oxide catalyst, both with and without manganese. US '266 also generally describes "high temperature catalysts" comprising one or more noble metals selected from Pt, Pd, Rh, Ir and Ru, as well as "low temperature catalysts" comprising at least one oxide or phosphate of a metal selected from Li, Mo, V, Nb, Sb, Sn, Zr, Mg, Mn, Ni, Co, Ce and rare earth metals (such as Sm), for oxidative dehydrogenation of alkanes. It is further stated that either type of catalyst may be promoted with additional metals and that any of the aforementioned catalyst compositions may be supported.

Mixed metal oxide catalysts having, as essential elements, Mo-Sb-W or Cr-Sb-W, and at least one metal selected from the group consisting of V, Nb, K, Mg, Sn, Fe, Co and Ni, were shown to be useful for oxidative dehydrogenation of propane to produce propene, in single-pass yields of greater than 10% (US Patent No. 6,239,325). A Pd-Cu/Mn catalyst on zirconium oxide support also catalyzed the oxidative dehydrogenation of ethane, with selectivities to ethane in the range of 70%-80% and diminished coke formation (US Patent Application Publication No. 2005/0124840).

The performance of vanadium-based catalysts for oxidative dehydrogenation of an alkane has been extensively investigated. For example, oxidative dehydrogenation of ethane in the presence of an Mo-V-Te-Nb-based mixed metal oxide catalyst has been shown to produce ethene in yields as high as 50%, and, in one case, even greater than 60% (US Patent Application Publication No. 2005/0085678). A vanadium-aluminum based mixed metal oxide catalyst, with or without one or more additional metal oxides of Cr, Zn, Fe and Mg, is known to be capable of catalyzing the conversion of propane, n-butane, isobutane, isopentane to their corresponding alkenes, in the presence of oxygen, to achieve relatively high alkene selectivities while minimizing the formation of coke and, thereby, minimizing the need for catalyst regeneration (US Patent No. 4,046,833). Oxidative dehydrogenation of ethane formed ethane in yields as high as 22% over a vanadium-phosphorous mixed oxide catalyst, which may be promoted with Co, U or W, as disclosed in US Patent No. 4,410,752. Zhaorigetu, et al. (1996) demonstrated that oxidative dehydrogenation of propane over an unsupported vanadium-based catalyst promoted with one or more rare earth metals (La, Ce, Pr, Sm and Er) could be enhanced by providing carbon dioxide, in addition to oxygen, to the reaction zone (Zhaorigetu, B.; Kieffer, R.; Hinderman J.-P., "Oxidative Dehydrogenation of Propane on Rare Earth Vanadates. Influence of the Presence of CO2 in the feed." Studies in Surface Science and Catalysis, 1996, 101, 1049-1058).

Since it is exothermic, when an oxidative dehydrogenation process is operated continuously, excess heat must be continuously removed, which increases capital and operating expenditures. Another disadvantage of oxidative dehydrogenation is that selectivity to alkene tends to decrease when the process is operated at higher, commercially useful alkane conversion rates. Thus, in practice, these processes tend to be operated at lower conversion rates (well below 100%), which limits their product yield capacity and generally renders them economically unsuitable for use in commercial-scale processes.

Other catalysts are known to catalyze the endothermic dehydrogenation of an alkane, in the presence of a "weak" oxidant, such as steam or carbon dioxide, to form the corresponding alkene. Some endothermic dehydrogenation catalysts perform better in the absence of oxygen, while others tolerate the presence of minor amounts of oxygen, along with the weak oxidant, without significant loss of activity.

For example, as discussed in the background section of US Patent No. 2,500,920, it has been long-known that a chromium oxide catalyst on an aluminum oxide support will catalyze dehydrogenation of n-butane to n-butene. US Patent No. 2,500,920 discloses that an aluminum oxide-based catalyst, with at least one oxide selected from the group consisting of molybdenum oxide, tungsten oxide, and vanadium oxide, and with chromium oxide as an activator, is capable of catalyzing the dehydrogenation of each of butane and pentane to their corresponding alkenes, respectively, in the presence of steam, without oxygen.

More recently, it has been shown that catalysts comprising, in particular, chromium (III) oxide ($Cr_2O_3$) are useful for catalytic dehydration of isobutane, with or without oxygen, to form isobutene (US Patent No. 5,013,706) with yields of about 30% to 40%. In US Patent Application Publication No. 2004/0181107, it was reported that chromium-based dehydrogenation catalysts, supported or unsupported, successfully catalyze endothermic dehydrogenation of isobutane to isobutene, in the presence of carbon dioxide, without oxygen, thereby avoiding coking problems, enhancing selectivity to isobutene, and achieving isobutene yields of up to 47%. A silicon-oxide-supported catalyst, comprising chromium oxide and an oxide of at least one metal selected from K, Mg, Ca, Ba, Ni, La or Fe, will successfully dehydrogenate propane to propene in the presence of carbon dioxide, as reported in Chinese Patent Application Publication No. 1472005, filed February 4, 2004.

Even more recently, supported vanadium-based catalysts, promoted with Li, Na, K or Mg, have been shown to dehydrogenate ethylbenzene in the presence of a "soft oxidant," i.e., carbon dioxide, to produce styrene with selectivities of about 98-99%, in the absence of oxygen (Li, X.-H.; Li, W.-Y.; Xie, K.-C., "Supported Vanadia Catalysts for Dehydrogenation of Ethylbenzene with CO2," Catalyst Letters, December 2005, Vol. 105, Nos. 3-4). Carbon dioxide was provided in varying amounts by Dury, et al. in 2002 to the oxidative dehydrogenation of propane to form propene in the presence of nickel-molybdenum-based catalysts, and found to increase conversion (by about 18 - 28%) but decrease selectivity (Dury, F.; Gaigneaux, E.M., Ruiz, P., "The Active Role of CO2 at Low Temperature in Oxidation Processes: The Case of the Oxidative Dehydrogenation of Propane on NiMoO4 catalysts," Applied Catalysis A: General 242 (2003), 187-203). Dury et al. demonstrated that, contrary to the traditional expectation that carbon dioxide is inert in dehydrogenation reactions, carbon dioxide actively participates in the dehydrogenation of propane, even in the absence of oxygen.

Takehira, et al. tested the activities of various metal oxide catalysts (Cr, Ga, Ni, V, Fe, Mn and Co) supported on silicon-containing support materials, including mesoporous MCM-41, Cab-O-Sil, and silicon oxide, and found that the Cr-based catalyst supported on MCM-41 provided the best results for dehydrogenation of propane, in the presence of carbon dioxide, to form propene. Takehira, K.; Oishi, Y.; Shishido, T.; Kawabata, T.; Takaki, K.; Zhang, Q.; and Wang, Y., "CO2 Dehydrogenation of Propane over Cr-MCM-41 Catalyst," Studies in Surface Science and Catalysis, 2004, 153, 323-328.

Obviously, endothermic dehydrogenation processes require addition of heat to the process. They typically involve burning (i.e., combusting) a hydrocarbon fuel, often different than the alkane to be dehydrogenated, with oxygen in a furnace or other vessel, resulting in increased costs due to increased initial capital investment and ongoing fuel consumption.

European Patent Application Publication No. EP 1112241 ("EP '241") describes a process designed to address this issue. Rather than burning a separate fuel to produce heat, the disclosed process involves combusting a portion of the alkane which is to be dehydrogenated, with oxygen, in the presence of a suitable combustion catalyst, to produce a heated stream containing the products of combustion (i.e., carbon oxides and water), unconsumed oxygen and unconsumed alkane. The heated stream is fed directly to an endothermic catalytic dehydrogenation reaction stage where the unreacted alkane is converted to the corresponding alkene in the presence of a suitable dehydrogenation catalyst.

More recently, International Patent Application Publication No. WO 2004/054945 ("WO '945") provides an improvement to the aforesaid two-stage exothermic-endothermic process, which eliminates the need for the combustion catalyst by substituting an ignition source, such as a pilot flame or a spark ignition, and burning a portion of the alkane to produce a heated stream comprising unreacted alkane, and either products of combustion (i.e., carbon oxides, water and heat), or synthesis gas (i.e., carbon monoxide and hydrogen).

Thus, in the processes of both EP '241 and WO '945, the need to burn a separately provided hydrocarbon fuel to preheat the alkane feed is avoided. However, a portion of the alkane reactant is consumed, which leaves less available for conversion to the desired product in the dehydrogenation stage. Furthermore, products of combustion are incidentally formed, which increases the amount of unwanted by-products, without any contribution to the quantity of the desired alkene product. In fact, when a portion of the alkane reactant itself is burned, as taught by these sources, a diminished amount of alkane remains available for the dehydrogenation reaction and less of the desired alkene product is produced.

Accordingly, notwithstanding the work conducted to date in this field, industry continues to grapple with the aforesaid problems of increasing overall production of alkene (i.e., increasing alkene selectivity and yield), while minimizing the costs of dehydrogenation of lower alkanes to their corresponding alkenes. Development of an improved process and catalyst system for converting an alkane to its corresponding alkene, which provide improved selectivity and yield of the desired product alkene would be welcomed by industry. It is believed that the processes and catalysts of the present invention address these needs.

## Summary of the Invention

The present invention provides a process for catalytic conversion of a $C_2$-$C_4$ alkane to its corresponding $C_2$-$C_4$ alkene which is thermally integrated and achieves better performance than any of the individual process components operated alone. More particularly, the process comprises A) contacting a $C_2$-$C_4$ alkane and oxygen with an upstream catalyst in an exothermic reaction zone, wherein the upstream catalyst is catalytically active for the exothermic conversion of the $C_2$-$C_4$ alkane to its corresponding $C_2$-$C_4$ alkene, in the presence of oxygen and B) exothermically converting a portion of the $C_2$-$C_4$ alkane to the corresponding $C_2$-$C_4$ alkene, in the exothermic reaction zone, to produce a heated mixed product gas which comprises the corresponding $C_2$-$C_4$ alkene, unreacted $C_2$-$C_4$ alkane, and heat produced by said exothermically converting step. The process further comprises contacting the heated mixed product gas and a weak oxidant with a downstream catalyst in an endothermic reaction zone, wherein the downstream catalyst is catalytically active for the endothermic conversion of the unreacted $C_2$-$C_4$ alkane to the corresponding $C_2$-$C_4$ alkene, in the presence of the weak oxidant and D) endothermically converting at least a portion of the unreacted $C_2$-$C_4$ alkane to the corresponding $C_2$-$C_4$ alkene, in the endothermic reaction zone, to produce a cumulative product stream which comprises at least the corresponding $C_2$-$C_4$ alkene produced in each of the reaction zones.

The upstream catalyst may be an oxidative dehydrogenation catalyst and the downstream catalyst may be an endothermic dehydrogenation catalyst. The oxidative dehydrogenation catalyst may be one or more of the following catalyst compositions: A) a catalyst comprising one or more noble metals selected from Pt, Pd, Rh, Ir and Ru; and B) a catalyst comprising at least one oxide of a metal selected from Li, Mo, W, V, Nb, Sb, Sn, Ga, Zr, Mg, Mn, Ni, Co, Ce and rare earth metals. For example, the oxidative dehydrogenation catalyst may comprise, as essential materials, vanadium oxide and at least one oxide of a metal selected from the group consisting of: niobium, copper and chromium. The endothermic dehydrogenation catalyst may be one or more of the following catalyst compositions: A) a catalyst comprising chromium oxide and, optionally, oxides of at least one metal selected from the group consisting of Mo, W, V, Ga, Mg, Ni, Fe, alkali elements, alkali earth elements, and rare earth elements; B) a catalyst comprising vanadium oxide and, optionally, at least one element selected from the group consisting of Li, Na, K and Mg; C) a catalyst comprising platinum and, optionally, at least one metal selected from the group consisting of sodium, potassium, cesium, rhenium and tin; and D) a catalyst comprising at least one metal selected from the group consisting of Ga, Fe, Mn and Co. For example, the endothermic dehydrogenation catalyst comprises, as essential materials, vanadium oxide, chromium oxide, and at least one metal selected from the group consisting of: copper, silver and gold.

Either or both of the oxidative dehydrogenation catalyst and the endothermic dehydrogenation catalyst may include a support material, such as alumina, silica, zirconia, titania, zeolites, rare earth metal oxides, other metal oxides, mesoporous materials, refractory materials, and combinations thereof. Either or both of the catalysts may further comprise a carrier, such as a monolithic carrier comprised of, for example, cordierite, metal, or ceramic.

In one embodiment of the present invention, the upstream oxidative dehydrogenation catalyst exothermically converts propane to propene in the presence of an oxygen-containing gas, and the downstream endothermic dehydrogenation catalyst endothermically converts propane to propene in the presence of a weak oxidant, such as carbon dioxide. The cumulative propene product may the then be fed to downstream partial oxidation processes wherein the propene is converted first to acrolein and then to acrylic acid.

## Brief Description of the Drawings

A more complete understanding of the present invention will be gained from the embodiments discussed hereinafter and with reference to the accompanying drawing, wherein:

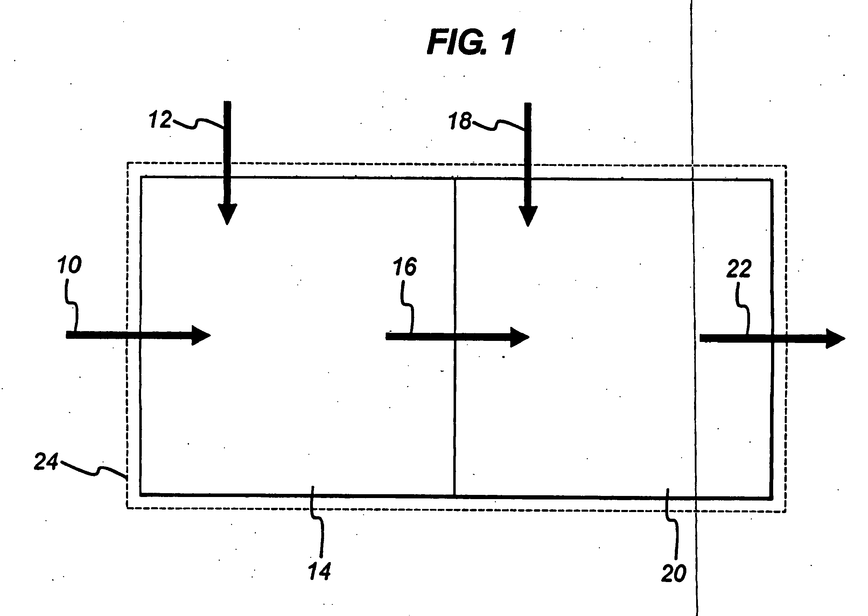

Figure 1 is a schematic representation of one embodiment of the process of the present invention.

Detailed Description of the Invention

The present invention provides a process for catalytic conversion of a $C_2$-$C_4$ alkane to its corresponding $C_2$-$C_4$ alkene. The process involves an upstream exothermic reaction which converts a $C_2$-$C_4$ alkane to its corresponding $C_2$-$C_4$ alkene and produces heat, and a downstream endothermic reaction which receives the product stream and heat from the exothermic reaction and converts the same $C_2$-$C_4$ alkane to the same corresponding $C_2$-$C_4$ alkene. The reactions are performed in sequence and are integrated so that the heat produced incidentally in the exothermic reaction is subsequently utilized in the endothermic reaction, minimizing the need for additional fuel and increasing the overall selectivity to, and yield of, the product alkene. Catalysts suitable for use in each of the dehydrogenation stages are also provided by the present invention.

The following definitions and meanings are provided for clarity and will be used hereinafter.

The term "hydrocarbon" means a compound which comprises at least one carbon atom and at least one hydrogen atom.

As used herein, the term "$C_2$ to $C_4$ alkane" means a straight chain or branched chain alkane having from 2 to 4 carbons atoms per alkane molecule, for example, ethane, propane and butane, which are typically in the vapor phase at ordinary temperatures and pressures (e.g., at least 10°C and 1 atmosphere). Accordingly, the term "$C_2$ to $C_4$ alkene" means a straight chain or branched chain alkene having from 2 to 4 carbons atoms per alkene molecule, for example, ethane, propene and butene.

The term "corresponding $C_2$-$C_4$ alkene" means the alkene having the same number of carbon atoms per alkene molecule as the alkane under discussion.

Furthermore, as used herein, the term "$C_2$ to $C_4$ alkanes and alkenes" includes at least one of the aforesaid $C_2$-$C_4$ alkanes, as well as its corresponding $C_2$-$C_4$ alkene. Similarly, when used herein in conjunction with the terms "$C_2$ to $C_4$ alkane", or "$C_2$ to $C_4$ alkene", or "$C_2$ to $C_4$ alkanes and alkenes", the terminology "a mixture thereof," means a mixture that includes at least one of the aforesaid alkanes having from 2 to 4 carbons atoms per alkane molecule, and the alkene having the same number of carbons atoms per alkene molecule as the alkane under discussion, for example, without limitation, a mixture of propane and propene, or a mixture of n-butane and n-butene.

An "inert" material, sometimes also referred to as a "diluent," is any material which is substantially inert, i.e., does not participate in, is unaffected by, and/or is inactive, in the particular reaction of concern. For example, nitrogen is inert in reactions that convert alkanes to their corresponding alkenes. As a more specific example, nitrogen is inert in oxidative dehydrogenation reactions that produce propene from propane. In the context of catalysts, where a mixed metal oxide catalyst useful in oxidation reactions is supported by a zirconium-based material, the zirconium-based material is considered to be inert and, therefore, does not directly affect, and is not directly affected by, the oxidation reaction being catalyzed by the mixed metal oxide catalyst. (Rather, without being bound by theory, it is believed that some support materials, such as zirconium, directly interact with the catalyst, which in turn may affect the conversion, selectivity, etc., of the oxidation reaction.)

The efficacy of chemical reaction processes, including those discussed herein, may be characterized and analyzed using the terms "feed conversion," "selectivity" to a particular product, and "product yield." These terms are used hereinafter and will have the following standard meanings.

The feed conversion, or simply "conversion", is the percentage of the total moles of feed (e.g., $C_3$ to $C_5$ alkanes and alkenes, such as propane and propene, or a mixture thereof) that have been consumed by the reaction, regardless of what particular products were produced, and is generally calculated as follows:

$$\text{feed conversion (\%)} = \frac{\text{moles of feed converted}}{\text{moles of feed supplied}} \times 100$$

The selectivity to a particular product, or simply "selectivity," is the percentage of the percentage of the total moles of feed (e.g., $C_3$ to $C_5$ alkanes, such as ethane, propane, and propene, or a mixture thereof) that have been consumed by the reaction, i.e., the portion of the feed that has been consumed was actually converted to the desired product, regardless of other products. Selectivity is generally calculated as follows:

$$\text{selectivity (\%)} = \frac{\text{moles of desired product produced}}{\text{moles of feed converted}} \times \frac{\text{number of carbon atoms in product}}{\text{number of carbon atoms in feed}} \times 100$$

The product yield, or simply "yield," is the percentage of the theoretical total moles of the desired product (alkene) that would have been formed if all of the feed had been converted to that product (as opposed to unwanted side products, e.g. acetic acid and $CO_x$ compounds), and is generally calculated as follows:

$$\text{product yield (\%)} = \frac{\text{moles of product produced}}{\text{moles of feed supplied}} \times \frac{\text{number of carbon atoms in product}}{\text{number of carbon atoms in feed}} \times 100$$

The term "oxygen-containing gas," as used herein, means any gas comprising from 0.01 % up to 100% oxygen or oxygen-containing compounds, including for example, without limitation: air, oxygen-enriched air, nitrous oxide, nitrogen dioxide, pure oxygen, mixtures of pure oxygen or oxygen-containing compounds with at least one inert gas, such as nitrogen, and mixtures thereof. Although the oxygen containing gas may be pure oxygen gas, it is usually more economical to use an oxygen containing gas, such as air, when purity is not particularly required.

"Oxidative dehydrogenation," as used herein, means a chemical reaction in which a hydrocarbon and oxygen are reacted to result in removal of one or more hydrogen atoms from the hydrocarbon to produce oxidation products. Thus, as this term is used herein, oxidative dehydrogenation requires an oxygen-containing gas or a gaseous oxygen-containing compound to provide the required oxygen.

"Exothermic oxidative dehydrogenation," as used herein, means an oxidative dehydrogenation process which produces heat in addition to oxidation product compounds.

"Endothermic dehydrogenation," as used herein, means a chemical reaction in which one or more hydrogen atoms are removed from a hydrocarbon, and which consumes heat, and so requires heat to be supplied from a source outside the reaction.

Endpoints of ranges are considered to be definite and are recognized to incorporate within their tolerance other values within the knowledge of persons of ordinary skill in the art, including, but not limited to, those which are insignificantly different from the respective endpoint as related to this invention (in other words, endpoints are to be construed to incorporate values "about" or "close" or "near" to each respective endpoint). The range and ratio limits, recited herein, are combinable. For example, if ranges of 1-20 and 5-15 are recited for a particular parameter, it is understood that ranges of 1-5, 1-15, 5-20, or 15-20 are also contemplated and encompassed thereby.

The process of the present invention involves sequential, thermally integrated reactions, each of which converts a particular $C_2$-$C_4$ alkane to the same corresponding $C_2$-$C_4$ alkene. This process produces a greater total yield of the corresponding $C_2$-$C_4$ alkene, with greater overall thermal efficiency, than either reaction alone. The process, as well as catalysts suitable for use therein, will first be described in general. Then a more detailed description is provided of an exemplary embodiment of the present invention, which is a combination of an oxidative dehydrogenation reaction and an endothermic dehydrogenation reaction for converting propane to propene. Notwithstanding the specificity of the exemplary embodiment, it will be appreciated and understood by persons of ordinary skill that the present invention is applicable to other types of reactions and products, and is subject to modifications and alterations, as necessary and desired, according to the ordinary skill and general knowledge of persons practicing in the relevant art. For example, the process of the present invention may be easily adapted by skilled persons to dehydrogenate a variety of hydrocarbons such as isopropane, ethane and ethyl benzene.

Referring now to the schematic representation of the process of the present invention provided in Figure 1, generally, a $C_2$-$C_4$ alkane 10 and oxygen 12 are contacted with an upstream catalyst (not shown per se) in an exothermic reaction zone 14. The upstream catalyst is catalytically active for the exothermic conversion of the $C_2$-$C_4$ alkane to its corresponding $C_2$-$C_4$ alkene in the presence of oxygen. The oxygen 12 may be supplied in the form of an oxygen-containing gas, as is well-known by persons of ordinary skill. The $C_2$-$C_4$ alkane 10 and oxygen 12 may be supplied to the exothermic reaction zone 14 separately and simultaneously, (as shown in Figure 1 ), or they may be blended together (not shown) and the resulting blended stream supplied to the exothermic reaction zone 14. One or more inert materials, or diluents (not shown), may also be provided to the exothermic reaction zone 14, separately or mixed with either, or both, of the $C_2$-$C_4$ alkane 10 and oxygen 12. Suitable diluents include, but are not limited to nitrogen, carbon dioxide, noble gases and steam. The feed composition to the exothermic reaction zone 15 may be, for example, 5-50vol% alkane, 2-30vol% oxygen, 0-50vol% carbon dioxide, and the remainder nitrogen, based upon the total volume of the feed materials. Another example of a suitable feed composition for the endothermic reaction zone may be, without limitation, 5-20vol% alkane, 2-15vol% oxygen, 10-40vol% carbon dioxide, and the remainder nitrogen, based upon the total volume of the feed materials.

A portion of the $C_2$-$C_4$ alkane is exothermically converted in the exothermic reaction zone 14, producing a heated mixed product gas 16 comprising at least the corresponding $C_2$-$C_4$ alkene, unreacted $C_2$-$C_4$ alkane, and heat produced by the exothermic conversion of the aforesaid portion of the $C_2$-$C_4$ alkane. The heated mixed product gas 16 may further comprise compounds including, but not limited to, carbon monoxide, carbon dioxide, other carbon-containing compounds, and water.

With reference still to Figure 1, the process of the present invention further comprises contacting the heated mixed product gas 16 and a mild oxidant, such as carbon dioxide 18, with a downstream catalyst (not shown per se) in an endothermic reaction zone 20. The downstream catalyst is catalytically active for the endothermic conversion of the unreacted $C_2$-$C_4$ alkane to the same corresponding $C_2$-$C_4$ alkene as produced in the exothermic reaction zone 14. Carbon dioxide 18 may be supplied to the endothermic reaction zone 20 in any manner known to persons of ordinary skill in the art. For example, as shown in Figure 1, carbon dioxide 18 may be provided as a separate stream directly to the endothermic reaction zone 20, simultaneously with the heated mixed product gas 16. Other options, which are not shown here, include, but are not limited to: blending the carbon dioxide 18 with the heated mixed product gas 16 before entry into the endothermic reaction zone 20, or blending the carbon dioxide 18 with one or more of the feed streams to the exothermic reaction zone 14 (i.e., with one or both of the $C_2$-$C_4$ alkane 10 and oxygen 12). One or more inert materials, or diluents (not shown), may also be provided to the endothermic reaction zone 20, separately or mixed with either, or both, of the heated mixed product gas 16 and carbon dioxide 18. Suitable diluents include, but are not limited to nitrogen, noble gases and steam. The feed composition to the endothermic reaction zone 20 may be, for example, 5-50vol% alkane, 0-5vol% oxygen, 10-80vol% carbon dioxide, and the remainder nitrogen, based upon the total volume of the feed materials. Another example of a suitable feed composition for the endothermic reaction zone may be, without limitation, 5-20vol% alkane, 0-2vol% oxygen, 20-60vol% carbon dioxide, and the remainder nitrogen, based upon the total volume of the feed materials.

At least a portion of the unreacted $C_2$-$C_4$ alkane is endothermically converted, in the endothermic reaction zone 20, to produce a cumulative product stream 22 which comprises at least the corresponding $C_2$-$C_4$ alkene formed in each of the exothermic and endothermic reaction zones 14, 20. The cumulative product stream 22 may also comprise one or more of the following compounds: unreacted $C_2$-$C_4$ alkane, unreacted oxygen, unreacted carbon dioxide, as well as other compounds including, but not limited to, carbon monoxide, water vapor, and hydrogen.

Although not shown in Figure 1, it will be readily recognized by persons of ordinary skill that the cumulative product stream 22 may be subjected to further processing and/or participate in additional reactions. For example, the cumulative product stream 22 may be supplied directly to another reaction process, such as vapor phase oxidation of the alkene to produce unsaturated carboxylic acids or nitriles. The cumulative product stream 22 may be further processed to purify the desired corresponding $C_2$-$C_4$ alkene product by separating at least a portion of unreacted reactants and other compounds from the cumulative product stream 22.

Determination of the quantities and how to supply the reactant materials ($C_2$-$C_4$ alkane, oxygen, carbon dioxide, etc.) to each of the exothermic and endothermic reaction zones is well within the ability of persons of ordinary skill in the art, based upon the knowledge generally available as well as the particular reactions, the desired products, and the catalysts selected for use in the reaction zones. For example, where carbon dioxide is expected to interfere with the performance of the selected upstream catalyst, then the carbon dioxide should not be blended with the initial reactant materials ($C_2$-$C_4$ alkane and oxygen) supplied to the exothermic reaction zone, but rather, the carbon dioxide should be blended with the heated mixed product gas, or supplied directly to the endothermic reaction zone. Conversely, where it is not possible to supply the carbon dioxide to the endothermic reaction zone, either directly or blended with the heated mixed product gas, then an upstream catalyst should be selected which can tolerate the presence of carbon dioxide. Where the performance of the downstream catalyst would be adversely affected by the presence of oxygen, the operating conditions in the oxidative reaction zone (e.g., temperature and bed size) may be adjusted for total consumption of the oxygen and, consequently, the heated mixed product gas provided to the endothermic reaction zone will be suitably oxygen free.

Catalysts suitable for use in the process of the present invention are not particularly limited and are generally known in the art. Suitable upstream and downstream catalysts are simply those which are catalytically active for the particular reaction that is to occur in each of the exothermic and endothermic reaction zones, respectively.

The upstream and downstream catalysts may be prepared by any suitable method known in the art, now or in the future. For example, the catalyst can be prepared by incipient wetness impregnation, chemical vapor deposition, hydrothermal synthesis, salt melt method, co-precipitation, and other methods. As will be discussed in further detail hereinafter, catalysts which are active for exothermic or endothermic conversion of $C_2$-$C_4$ alkanes to produce the corresponding $C_2$-$C_4$ alkenes typically comprise one or more metals and/or metal oxides. In addition, either or both of the upstream and downstream catalysts may be promoted, for example, with suitable metals or metal oxides.

Furthermore, either or both of the upstream and downstream catalysts may further comprise support material. The catalyst materials may be applied to the support by any method known in the art and at any time including, but not limited to, during preparation of the catalyst material, before or after calcination, and even before or after addition of a promoter. Typical and suitable support materials include, but are not limited to: magnesium oxide, zirconia, stabilized zirconia, zirconia stabilized alumina, yttrium stabilized zirconia, calcium stabilized zirconia, alumina, titania, silica, magnesia, nitrides, silicon carbide, cordierite, cordierite-alpha alumina, alumina-silica magnesia, zircon silicate, magnesium silicates, calcium oxide, silica-alumina, alumina-zirconia, alumina-ceria, and combinations thereof. Additionally, suitable catalyst supports may comprise rare earth metal oxides, mixed metal oxides, mesoporous materials, refractory materials, and combinations thereof. The support may be modified, stabilized, or pretreated in order to achieve the proper structural stability desired for sustaining the operating conditions under which the catalysts will be used.

The support can be in the shape of wire gauzes, monoliths, particles, honeycombs, rings, and others. Where the support is in the form of particles, the shape of the particles is not particularly limited and may include granules, beads, pills, pellets, cylinders, trilobes, spheres, irregular shapes, etc.

Monoliths typically comprise any unitary piece of material of continuous manufacture, such as, for example, pieces of metal or metal oxide, foam materials, or honeycomb structures. It is known in the art that, if desired, a reaction zone may comprise two or more such catalyst monoliths stacked upon one another. For example, the catalyst can be structured as, or supported on, a refractory oxide "honeycomb" straight channel extrudate or monolith, made of cordierite or mullite, or other configuration having longitudinal channels or passageways permitting high space velocities with a minimal pressure drop.

Furthermore, the catalyst material may be deposited as washcoats on the monolithic support by methods known to people skilled in the art. Additionally, catalyst material may be combined with the monolithic support by depositing the support material as washcoats and, successively, impregnating the support material washcoats with the active catalyst material, such as, without limitation, vanadium oxide or platinum, followed by calcination of the combined support and catalyst materials.

Monolithic supports may comprise stabilized zirconia (PSZ) foam (stabilized with Mg, Ca or Y), or foams of $\alpha$-alumina, cordierite, ceramics, titania, mullite, zirconium-stabilized $\alpha$-alumina, or mixtures thereof. Monolithic supports may also be fabricated from metals and their alloys, such as, for example, aluminum, steel, fecralloy, hastalloy, and others known to persons skilled in the art. Additionally, other refractory foam and non-foam monoliths may serve as satisfactory supports. The promoter metal precursor and any base metal precursor, with or without a ceramic oxide support forming component, may be extruded to prepare a three-dimensional form or structure such as a honeycomb, foam or other suitable tortuous-path or straight-path structure.

As shown in Figure 1, the exothermic and endothermic reaction zones 14, 20 may be contained in a single reactor 24 (shown in phantom), which may be any suitable reactor known in the art including, but not limited to, a batch reactor, a stirred tank reactor, a continuous stirred tank reactor (CSTRs), a tubular reactor, a shell-and-tube heat exchanger reactor, a multiple-pass reactor, a reactor having microchannels, a short contact time reactor, a catalytic fixed bed reactor, and a reactor having a combination of the foregoing features. Each reaction zone 14, 20 may, instead, be disposed within separate reactors (not shown), and various combinations of reactors and reaction zones may be arranged. Each reaction zone 14, 20 may or may not include sub-zones (also not shown), which differ by operating temperature, or catalyst composition, or catalyst concentration, or in other ways which are known to persons of ordinary skill. Furthermore, the upstream and downstream catalysts may be configured in their respective reaction zones in any suitable arrangement including, but not limited to, a fixed bed, a fluidized bed, and a spouted bed. All such configurations are well known in the art.

As discussed in further detail hereinafter in connection with an exemplary embodiment, it is within the ability of persons of ordinary skill in the relevant art to select appropriate operating conditions for each of the exothermic and endothermic reaction zones, depending on the particular products desired and the reactions and catalysts selected to produce the desired product.

In an exemplary embodiment, the upstream catalyst may be an oxidative dehydrogenation catalyst (ODH catalyst) which catalyzes the exothermic catalytic oxidative dehydrogenation of a $C_2$-$C_4$ alkane with oxygen, to produce the corresponding $C_2$-$C_4$ alkene and heat. Furthermore, the upstream catalyst may, for example, be contained in a fixed bed, tubular reactor.

Persons of ordinary skill will be familiar with various ODH catalysts that may be successfully used in the exothermic reaction zone, in accordance with process of the present invention. Suitable categories of ODH catalysts include, but are not limited to: catalysts comprising one or more noble metals selected from Pt, Pd, Rh, Ir and Ru; and catalysts comprising at least one oxide of a metal selected from Li, Mo, W, V, Nb, Sb, Sn, Ga, Zr, Mg, Mn, Ni, Co, Ce and rare earth metals. For example, mixed metal oxide catalysts having, as essential elements, Mo-Sb-W or Cr-Sb-W, and at least one metal selected from the group consisting of V, Nb, K, Mg, Sn, Fe, Co and Ni; as well as vanadium-aluminum-based mixed metal oxide catalysts, with or without one or more additional metal oxides of Nb, Cr, Zn, Fe, Mo, Mg and rare earth elements; and, furthermore, vanadium-based catalysts promoted with one or more of La, Ce, Pr, Sm and Er, have all been shown to catalyze the exothermic oxidative dehydrogenation of propane to form propene. In an exemplary embodiment, the ODH catalyst comprises, as essential materials, vanadium oxide and at least one oxide of a metal selected from the group consisting of: niobium, magnesium, molybdenum and rare earth elements As already mentioned, the ODH catalysts may be supported by materials such as alumina, platinum, silica, other metal oxides, microporous materials, mesoporous materials, and refractory materials. For example, a vanadium-niobium-oxide catalyst may be combined and supported on a silicon oxide support material and advantageously used in the exothermic reaction zone of the process of the present invention.

Applicants have surprisingly found that high activity ODH catalysts, such as vanadium-oxide-based catalysts supported on certain materials such as alumina, zirconia, or titania (as opposed to, for instance, silica), will catalyze the exothermic ODH conversion of propane to propene better if the supported catalyst is loaded onto a monolithic carrier, rather than if used alone in particulate (powder) form. For example, a silica-supported vanadium-based ODH catalyst was found to provide similar performance for ODH of propane, regardless of whether deposited on a cordierite monolith as washcoats, or simply used in powder form (having average particulate size of 300-500 microns). However, surprisingly, an alumina-supported vanadium-based catalyst composition loaded onto a cordierite monolith carrier as washcoats performed better for ODH of propane, than when used in powder form (also having average particulate size of 300-500 microns). Furthermore, similar performance improvements are expected even when the monolithic carrier is formed from materials other than cordierite, such as, for example, metal or ceramic, in the form of any unitary body including, but not limited to a monolith, a honeycomb, a plate, foam, heat exchanger components, reactor chamber walls, etc.

Suitable operating conditions for oxidative dehydrogenation of a $C_2$-$C_4$ alkane in the exothermic reaction zone are generally known in the art and determinable by persons of ordinary skill. For example, the $C_2$-$C_4$ alkane, oxygen and, optionally, a diluent, are typically supplied to the exothermic reaction zone, separately or in various combinations with one another, at a total gas hourly space velocity (GHSV) of between about 1,000 $hr^{-1}$ to 1,000,000 $hr^{-1}$, for example, between about 5,000 $hr^{-1}$ and 200,000 $h^{-1}$, or even 5,000 $hr^{-1}$ to 10,000 $hr^{-1}$. The reaction pressure is typically in the range of from 0.1 to about 10 atmospheres (atm), for example, from 0.8 to 5.0 atm; and the reaction temperature is typically maintained between 400°C and 1,100°C, for example, between 450°C and 650°C. Contact time between the reactants and catalyst is typically in the range of from 10 milliseconds (ms) (360,000 $h^{-1}$) to 4 seconds (900 $h^{-1}$). The molecular ratio of $C_2$-$C_4$ alkane to oxygen supplied to the exothermic reaction zone may, for example, be in a range of from 1:1 to 10:1, such as between 1:1 and 5:1.

In this exemplary embodiment, when the selected upstream catalyst is an ODH catalyst, the downstream catalyst may be an endothermic dehydrogenation catalyst which catalyzes the endothermic dehydrogenation of the same $C_2$-$C_4$ alkane, in the presence of a mild oxidant, to the same corresponding $C_2$-$C_4$ alkene as the ODH catalyst in the exothermic reaction zone. For example, if the upstream catalyst is an oxidative dehydrogenation catalyst which converts propane to propene, then the downstream catalyst may be an endothermic dehydrogenation catalyst which also converts propane to propene. The mild oxidant may be, for example, without limitation, carbon dioxide, steam, or a combination thereof. As discussed hereinabove, many such endothermic dehydrogenation catalysts are known and would be suitable for use the endothermic reaction zone in accordance with process of the present invention.

Persons of ordinary skill will be familiar with various endothermic dehydrogenation catalysts that may be successfully used in the endothermic reaction zone, in accordance with process of the present invention. Suitable categories of endothermic dehydrogenation catalysts include, but are not limited to: chromium oxide-based catalysts, which may also comprise oxides of at least one metal selected from the group consisting of Mo, W, V, Ga, Mg, Ni, Fe, alkali elements, alkali earth elements, and rare earth elements; as well as vanadium oxide-based catalysts, which may be promoted with Li, Na, K or Mg; platinum-based catalysts which may also comprise at least one metal selected from the group consisting of sodium, potassium, cesium, rhenium and tin; and a catalyst comprising at least one metal selected from the group consisting of Ga, Fe, Mn and Co.

As will be easily recognized by skilled persons, there are many catalyst compositions suitable for use in the endothermic reaction zone in accordance with the present invention. For example, the endothermic dehydrogenation catalyst may comprise a vanadium-chromium-oxide, and, optionally, at least one oxide of a metal selected from the group consisting of: copper, silver, and gold. The vanadium-chromium-oxide catalyst may also comprise oxides of one or more metals selected from the group consisting of: Cu, Ag, Au, K, Cs, Pt, Rh, and additional metal oxides. Furthermore, the vanadium-chromium-oxide catalysts may be modified with one or more reagents selected from the group consisting of: phosphate and sulfate.

As already mentioned, the endothermic dehydrogenation catalysts may be combined and supported on materials such as alumina, platinum, silica, zirconia, zeolites, other metal oxides, microporous materials, mesoporous materials, and refractory materials. For example, silicon oxide material or mesoporous material, such as MCM-41, may be used to support a vanadium oxide-based catalyst, such as V/Cr/Si/O, V/Cr/Ag/Si/O, V/Cr/Ag/Cs/Si/O, and others. Additionally, other examples include, without limitation: a V/Cr/Mn/W/O catalyst supported on alumina, platinum oxide-based catalysts supported on a microporous zeolite material such as ZSM-5, and a V/Cr/AgO catalyst supported on gallium oxide material (e.g., $\beta$-$Ga_2O_3$). As will be easily recognized by skilled persons, there are many combinations of catalyst compositions and support materials that will be suitable for use in the endothermic reaction zone in accordance with the present invention.

Suitable operating conditions for endothermic dehydrogenation of a $C_2$-$C_4$ alkane are generally known by persons of ordinary skill and are applicable to operation of the endothermic reaction zone. For example, carbon dioxide, the heated mixed product gas comprising unreacted $C_2$-$C_4$ alkane and, optionally, a diluent, may be supplied to the endothermic reaction zone, separately or mixed, at a total gas hourly space velocity (GHSV) of about 500 $hr^{-1}$ to 100,000 $hr^{-1}$. The reaction pressure is typically in the range of from 0.1 to about 10 atm, for example, from 0.8 to 5.0 atm, and the reaction temperature is typically maintained between 300°C and 900°C, for example, between 450°C and 700°C. Contact time between the reactants and catalyst is typically in the range of from 36 ms (100,000 h$^{-1}$) to 7.2 seconds (500 h$^{-1}$), such as, for example, from 200 ms to 5 seconds. The molecular ratio of unreacted $C_2$-$C_4$ alkane to mild oxidant, such as carbon dioxide, supplied to the exothermic reaction zone may, for example, be in a range of from 1:0.1 to 1:10, or even between 1:1 and 1:5. It is noted that short contact time (SCT) operating conditions have been used, as an alternative to traditional steam cracking and non-oxidative dehydrogenation processes, to perform oxidative dehydrogenation of $C_2$-$C_4$ alkenes, wherein the contact time of the reactants with the oxidative dehydrogenation catalyst is typically in the range of 1 to 650 ms, under temperatures of between 200°C and 1100°C, and pressures of from 0.3 atm to 40 atm.

## EXAMPLES

EXAMPLES OF ODH/DH INTEGRATED PROCESS

The catalyst selected for the (upstream) oxidative dehydrogenation catalyst, V/Nb/O$_x$ supported on silicon oxide, was prepared as follows.

Silica support material (Davisil grade 646) was impregnated to incipient wetness with a solution containing ammonium metavanadate and ammonium niobium oxalate in 1.3 molar aqueous oxalic acid solution providing 0.05 g $V_2O_s$ and 0.05g $Nb_2O_5$ per gram of catalyst. The material was dried at 120°C for 8 hours and calcined at 500°C for 2 hours.

The catalyst selected for the (downstream) endothermic dehydrogenation catalyst, V/Cr/Ag/O$_x$ supported on silicon oxide, was prepared as follows.

10 grams of silica support material (Merck Silica gel with an average surface are of 675 square meter per gram, pore volume of 0.68 cubic centimeters per gram, and an average pore diameter of 40 Angstroms (Å)) was impregnated to incipient wetness with a 7 millimeter homogeneous solution of chromium (III) nitrate hydrate (~13wt% Cr), vanadium (IV) sulfate hydrate (~21wt% V) and silver nitrate (~64wt% Ag). The resulting atomic ratio of chromium, vanadium and silver was 1:1:0.05. After 60 minutes of soaking in closed containment, the wet and blue silica precursor was placed in a ceramic dish covered loosely with a lead to provide suitable conditions for the steam calcination process. The calcination step was carried out in three steps: first heating to 80°C for 3 hours in isotherm box furnace. Following with drying step at 125°C for 6 hours, and finally calcination at 300°C for 1 hour then at 650°C for 2 hours with continuous air-purge internally at about 2-4 standard liters per minute (SLPM).

Apparatus Configuration Used For Examples 1 - 3

Conversion of propane by successive oxidative dehydrogenation and endothermic dehydrogenation (the "Hybrid Process") was carried out in a quartz tubular microreactor 0.75-inch (1.9 centimeter) outer diameter (OD) which comprised an upstream exothermic reaction zone and an adjacent downstream endothermic reaction zone. Reaction conditions were as follows: 500°C to 650°C, at 1-psig pressure (1.07 atm), and a flow rate of 620 standard cubic centimeters per minute (SCCM). The feed gas mixture comprised: 10% propane, 40 % carbon dioxide, 45% nitrogen, and 5% oxygen (Feed A). This feed was used for the independent evaluation of ODH catalysts as well as in the ODH/ED hybrid process experiments described herein below.

For the independent evaluation of endothermic dehydrogenation catalysts, the feed gas mixture comprised: 10% propane, 40 % carbon dioxide, and 50% nitrogen (Feed B). The online gas product analysis was performed using a gas chromatograph equipped with a flame ionization detector and a thermal conductivity detector (FID/TCD).

Example 1

2 milliliters (ml) of the V/Nb/Si/O$_x$ oxidative dehydrogenation catalyst prepared by the process described above, was disposed in the exothermic reaction zone, and 2 ml of the V/Cr/Ag/Si/O$_x$ endothermic dehydrogenation catalyst was used in the endothermic reaction zone. Each catalyst was independently tested at 625°C and at 0.4 sec residence time. While the catalyst in the 1$^{st}$ zone, V/Nb/Si/O$_x$, was evaluated with feed A, the catalyst in the 2$^{nd}$ zone, V/Cr/Ag/Si/O$_x$, was tested with feed B. The hybrid experiment (i.e., both catalysts) was carried out with feed A. The results of these of experiments are provided in Table 1. As shown, the cumulative yield for the Hybrid Process was greater than for either of the reaction zones operated separately.

Table 1: 625°C. 0.4 seconds residence time, with Feed A.

| Reaction Zone | Catalyst | T°C | C% | S% | Y% |
|---|---|---|---|---|---|
| ODH | V/Nb * | 625 | 30.3 | 55.8 | 16.9 |
| Endothermic | V/Cr/Ag** | 625 | 20.5 | 81.9 | 16.8 |
| Hybrid - Both | V/Nb & /Cr/Ag | 625 | 31.6 | 63.5 | 20.0 |
| Temperature= T°C; Conversion of Propane= C%; Selectivity to Propene= S%; Yield Propene=Y% <br> * Run independently with Feed A <br> ** Run independently with Feed B | | | | | |

The selected oxidative dehydrogenation catalyst, V/Nb/$O_x$ supported on silicon oxide, responded well to having carbon dioxide and oxygen in the feed gas mixture and generated heat while producing propylene in the exothermic reaction zone. The selected endothermic dehydrogenation catalyst, V/Cr/Ag/$O_x$ supported on silicon oxide, exhibited enhanced performance in the presence of carbon dioxide, without oxygen, and used the heat generated in the ODH reaction zone for supplementary propylene production in the endothermic reaction zone.

Example 2

The second experiment was conducted with the same catalysts, in the same quantities, as in Example 1, but at a residence time of 0.6 seconds. The results are provided in Table 2 below. As shown, the cumulative yield for the Hybrid Process was greater than for either of the reaction zones operated separately.

Table 2: 522°C. 0.6 seconds residence time, with Feed A

| Reaction Zone | Catalyst | T°C | C% | S% | Y% |
|---|---|---|---|---|---|
| ODH | V/Nb * + | 577 | 47.3 | 56.8 | 27.0 |
| Endothermic | V/Cr/Ag** | 582 | 25.9 | 92.7 | 24.0 |
| Hybrid - Both | V/Nb & /Cr/Ag | 522 | 36.7 | 83.9 | 31.0 |
| Temperature= T°C; Conversion of Propane= C%; Selectivity to Propene= S%; Yield Propene=Y% <br> * Run independently with feed A <br> ** Run independently with feed B | | | | | |

Example 3

The Hybrid Process may also be operated, in accordance with the present invention, using feed gas mixtures containing higher concentrations of propane than in Examples 1 or 2, such as from about 10% to about 50%.
The following feed gas mixtures are used:

Feed C: 40 % propane, 5% oxygen, 45% carbon dioxide, and 10% nitrogen
Feed D: 40 % propane, 50% carbon dioxide, and 10% nitrogen

The third experiment is conducted with the same catalysts, in the same quantities, and the same residence time as Example 1, but at a temperature of 522°C and using Feeds C and D. The expected results are provided in Table 3 below. It is expected that the cumulative yield for the Hybrid Process will be greater than for either of the reaction zones operated separately.

Table 3: 522°C, 0.6 seconds, with Feed C

| Reaction Zone | Catalyst | T°C | C% | S% | Y% |
|---|---|---|---|---|---|
| ODH | V/Nb * | 577 | 29.6 | 56.8 | 16.8 |
| Endothermic | V/Cr/Ag** | 582 | 27.5 | 85.3 | 23.5 |

(continued)

| Reaction Zone | Catalyst | T°C | C% | S% | Y% |
|---|---|---|---|---|---|
| Hybrid - Both | V/Nb & /Cr/Ag | 522 | 30.9 | 83.9 | 25.9 |

Temperature= T°C; Conversion of Propane= C%; Selectivity to Propene= S%; Yield Propene=Y%
* Run independently with feed C
** Run independently with feed D

EXAMPLES OF ODH WITH CATALYST ON MONOLITH OR POWDER SUPPORT

Apparatus Configuration Used For Examples 4-5

Performance of the supported catalysts in Examples 4 and 5, was carried out under oxidative dehydrogenation of propane in a quartz tubular microreactor 10 millimeter inner diameter.

Reaction conditions were as follows: 600°C, at 1-psig pressure, with reactant flow rates as indicated below. The feed gas mixture comprised: 10% propane, 5% oxygen, and 85% nitrogen. This feed was used for all of evaluation of all of the supported catalysts described below in Examples 4 and 5. Product analysis was performed using a gas chromatograph equipped with FID/TCD.

Example 4 - Vanadium-Silica Supported on Cordierite (Powder vs. Monolith)

A powder catalyst of composition 5wt% $V_2O_5/SiO_2$ was prepared by impregnating a sample of silica gel (Merck, grade 10181; 300-500 micron particle size) with a solution of ammoniummetavanadate in aqueous 1.3 molar oxalic acid, providing 0.05g $V_2O_5$ per gram of catalyst, after calcination. The material was dried at 120°C for 8 hours and calcined at 500°C for 2 hours. The catalyst powder (1 g) was loaded onto a quartz frit in the tubular reactor. The feed gas was provided at a flow rate of 0.5 SLPM.

A monolith catalyst of composition 5wt% $V_2O_5/SiO_2$ was prepared from the aforesaid 5%$V_2O_5/SiO_2$ powder catalyst by addition of water to form a slurry, milling the slurry particles to an average size of less than 10 microns, and then adding 10% by weight $SiO_2$ as colloidal silica solution (Ludox; Aldrich) as a binder. A washcoat of the V-Si powder and binder mix was deposited on a cordierite substrate (Corning; 62 cells per square centimeter and 1.65 mllimeter wall thickness) to a loading of 3 grams per cubic inch (0.183 grams per cubic centimeter). Monolith catalysts having 50 millimeter length and 9.5 millimeter outer diameter, were mounted in the tubular reactor using ceramic paper. The feed gas was provided at a flow rate of 0.25 SLPM.

Table 4: Powder and Monolith Cordierite-Supported V-Si Catalysts

| Sample | T°C | O$_2$% | C% | Y% |
|---|---|---|---|---|
| Powder $V_2O_5$/silica | 600 | 100 | 31.6 | 15.7 |
| Monolith $V_2O_5$/silica | 600 | 100 | 31.4 | 15.0 |

Temperature= T°C; Conversion of Oxygen= O$_2$%; Conversion of Propane= C%;; Yield Propene=Y%

Example 5 - Vanadium-Alumina Supported on Cordierite (Powder vs. Monolith)

A powder catalyst of composition 5wt% $V_2O_5/Al_2O_3$ was prepared by impregnating a sample of porous alumina (nitrogen pore volume of approximately 1.0 ml/gram and area of 145 square meters per gram) with a solution of ammoniummetavanadate in aqueous 1.3 molar oxalic acid, providing 0.05 gram $V_2O_5$ per gram of catalyst, after calcination. The material was dried at 120°C for 8 hours and calcined at 500°C for 2 hours. The catalyst powder (300-500 micron particle size; 0.125g+0.875g quartz) was loaded onto a glass frit in the tubular reactor. The feed gas was provided at a flow rate of 0.25 SLPM.

A monolith catalyst of composition 6.8wt% $V_2O_5/Al_2O_3$ was prepared by depositing an alumina washcoat, by standard washcoating technique, using pseudo-boehmite as the binder, on a cordierite substrate (Corning; 62 cells per square centimeter and 1.65 mllimeter wall thickness), to a loading of 1.4 grams per cubic inch. The alumina was subsequently impregnated with a solution of ammoniummetavanadate in aqueous 1.3 molar oxalic acid, providing 0.068 gram $V_2O_5$ per gram of alumina washcoat. The material was dried at 120°C for 8 hours and calcined at 700°C for 2 hours. Monolith catalysts having 19.1 millimeter length and 9.8 millimeter outer diameter, were mounted in the tubular reactor using ceramic paper. The feed gas was provided at a flow rate of 0.25 SLPM.

Table 5: Powder and Monolith Alumina-Supported Catalysts

| Sample | T°C | $O_2$% | C% | Y% |
|---|---|---|---|---|
| Powder $V_2O_5$/alumina | 600 | 100 | 27.5 | 10.5 |
| Monolith $V_2O_5$/alumina | 600 | 100 | 27.8 | 12.7 |
| Temperature= T°C; Conversion of Oxygen= $O_2$%; Conversion of Propane= C%;; Yield Propene=Y% | | | | |

**Claims**

1. A process for conversion of a $C_2$-$C_4$ alkane to its corresponding $C_2$-$C_4$ alkene, said process comprising the steps of:

A) contacting a $C_2$-$C_4$ alkane and oxygen with an upstream catalyst in an exothermic reaction zone, wherein the upstream catalyst is catalytically active for the exothermic conversion of the $C_2$-$C_4$ alkane to its corresponding $C_2$-$C_4$ alkene, in the presence of oxygen; and
B) exothermically converting a portion of the $C_2$-$C_4$ alkane to the corresponding $C_2$-$C_4$ alkene, in the exothermic reaction zone, to produce a heated mixed product gas which comprises the corresponding $C_2$-$C_4$ alkene, unreacted $C_2$-$C_4$ alkane, and heat produced by said exothermically converting step;
C) contacting the heated mixed product gas and a weak oxidant with a downstream catalyst in an endothermic reaction zone, wherein the downstream catalyst is catalytically active for the endothermic conversion of the unreacted $C_2$-$C_4$ alkane to the corresponding $C_2$-$C_4$ alkene, in the presence of the weak oxidant;
D) endothermically converting at least a portion of the unreacted $C_2$-$C_4$ alkane to the corresponding $C_2$-$C_4$ alkene, in the endothermic reaction zone, to produce a cumulative product stream which comprises at least the corresponding $C_2$-$C_4$ alkene produced in each of the reaction zones.

2. The process of Claim 1, wherein the upstream catalyst comprises an oxidative dehydrogenation catalyst.

3. The process of Claim 2, wherein the oxidative dehydrogenation catalyst comprises at least one catalyst composition selected from the group consisting of:

A) a catalyst comprising one or more noble metals selected from Pt, Pd, Rh, Ir and Ru; and
B) a catalyst comprising at least one oxide of a metal selected from Li, Mo, W, V, Nb, Sb, Sn, Ga, Zr, Mg, Mn, Ni, Co, Ce and rare earth metals.

4. The process of Claim 2, wherein the oxidative dehydrogenation catalyst comprises a support material.

5. The process of Claim 4, wherein the support material of the oxidative dehydrogenation catalyst comprises a material selected from the group consisting of: alumina, titanium, zirconium, silica, zeolites, rare earth metal oxides, mixed metal oxides, mesoporous materials, refractory materials, and combinations thereof.

6. The process of Claim 5, wherein the oxidative dehydrogenation catalyst comprises, as essential materials, vanadium oxide and at least one oxide of a metal selected from the group consisting of: niobium, magnesium, molybdenum and rare earth elements.

7. The process of Claim 5, wherein the support material of the oxidative dehydrogenation catalyst comprises silicon oxide.

8. The process of Claim 1, wherein the downstream catalyst comprises an endothermic dehydrogenation catalyst.

9. The process of Claim 8, wherein the endothermic dehydrogenation catalyst comprises at least one catalyst composition selected from the group consisting of:

A) a catalyst comprising chromium oxide and, optionally, oxides of at least one metal selected from the group consisting of Mo, W, V, Ga, Mg, Ni, Fe, alkali elements, alkali earth elements, and rare earth elements;

B) a catalyst comprising vanadium oxide and, optionally, at least one element selected from the group consisting of Li, Na, K and Mg;

C) a catalyst comprising platinum and, optionally, at least one metal selected from the group consisting of sodium, potassium, cesium, rhenium and tin; and

D) a catalyst comprising at least one metal selected from the group consisting of Ga, Fe, Mn and Co.

10. The process of Claim 8, wherein the endothermic dehydrogenation catalyst comprises a support material.

11. The process of Claim 10, wherein the support material of the endothermic dehydrogenation catalyst comprises a material selected from the group consisting of: alumina, titanium, zirconium, silica, zeolites, rare earth metal oxides, mixed metal oxides, mesoporous materials, refractory materials, and combinations thereof.

12. The process of Claim 11, wherein the endothermic dehydrogenation catalyst comprises, as essential materials, vanadium oxide, chromium oxide, and at least one metal selected from the group consisting of: copper, silver and gold.

13. The process of Claim 11, wherein the support material of the endothermic dehydrogenation catalyst comprises silicon oxide.

14. The process of Claim 8, wherein the weak oxidant comprises carbon dioxide.

15. The process of Claim 1, wherein an oxygen-containing gas is supplied to the exothermic reaction zone separately from the $C_2$-$C_4$ alkane.

16. The process of Claim 1, wherein at least a portion of the weak oxidant is supplied to the exothermic reaction zone separately from the $C_2$-$C_4$ alkane.

17. The process of Claim 1, wherein at least a portion the weak oxidant is supplied to the endothermic reaction zone separately from the heated mixed product gas.

18. The process of Claim 1, wherein the $C_2$-$C_4$ alkane comprises propane, the corresponding $C_2$-$C_4$ alkene comprises propene, and the weak oxidant comprises carbon dioxide.

**Patentansprüche**

1. Verfahren zur Umwandlung eines $C_2$-$C_4$-Alkans in sein entsprechendes $C_2$-$C_4$-Alken, wobei das Verfahren die Schritte umfaßt:

A) Inkontaktbringen eines $C_2$-$C_4$-Alkans und Sauerstoffs mit einem vorgeschalteten bzw. einem upstream-Katalysator in einem exothermen Reaktionsbereich, wobei der vorgeschaltete Katalysator für die exotherme Umwandlung des $C_2$-$C_4$ Alkans in sein entsprechendes $C_2$-$C_4$ Alken in Anwesenheit von Sauerstoff katalytisch aktiv ist, und

B) exothermes Umwandeln eines Teils des $C_2$-$C_4$ Alkans in das entsprechende $C_2$-$C_4$ Alken in dem exothermen Reaktionsbereich, um ein erwärmtes, gemischtes Produktgas herzustellen, welches das entsprechende $C_2$-$C_4$ Alken, nicht reagiertes $C_2$-$C_4$ Alkan und Wärme umfaßt, die durch den exothermen Umwandlungsschritt erzeugt wurde,

C) Inkontaktbringen des erwärmten, gemischten Produktgases und eines schwachen Oxidationsmittels mit einem nachgeschalteten bzw. einem downstream-Katalysator in einem endothermen Reaktionsbereich, wobei der nachgeschaltete Katalysator für die endotherme Umwandlung des nicht reagierten $C_2$-$C_4$ Alkans in das entsprechende $C_2$-$C_4$ Alken in Anwesenheit des schwachen Oxidationsmittels katalytisch aktiv ist,

D) endothermes Umwandeln zumindest eines Teils des nicht reagierten $C_2$-$C_4$ Alkans in das entsprechende $C_2$-$C_4$ Alken in dem endothermen Reaktionsbereich, um einen kumulativen Produktstrom zu erzeugen, der mindestens das entsprechende $C_2$-$C_4$ Alken, das in jedem der Reaktionsbereiche erzeugt wurde, umfaßt.

2. Verfahren nach Anspruch 1, wobei der vorgeschaltete Katalysator einen oxidativen Dehydrierungs-Katalysator umfaßt.

**3.** Verfahren nach Anspruch 2, wobei der oxidative Dehydrierungs-Katalysator mindestens eine Katalysatorzusammensetzung umfaßt, ausgewählt aus der Gruppe, bestehend aus:

A) einem Katalysator, umfassend ein oder mehrere Edelmetalle, ausgewählt aus Pt, Pd, Rh, Ir und Ru, und
B) einem Katalysator, umfassend mindestens ein Oxid eines Metalls, ausgewählt aus Li, Mo, W, V, Nb, Sb, Sn, Ga, Zr, Mg, Mn, Ni, Co, Ce und Seltenerdmetallen.

**4.** Verfahren nach Anspruch 2, wobei der oxidative Dehydrierungs-Katalysator ein Trägermaterial umfaßt.

**5.** Verfahren nach Anspruch 4, wobei das Trägermaterial des oxidativen Dehydrierungs-Katalysators ein Material umfaßt, ausgewählt aus der Gruppe, bestehend aus: Aluminiumoxid, Titan, Zirkon, Siliziumoxid, Zeolithen, SeltenerdmetallOxiden, gemischten Metalloxiden, mesoporösen Materialien, feuerfesten Materialien und Kombinationen davon.

**6.** Verfahren nach Anspruch 5, wobei der oxidative Dehydrierungs-Katalysator als essentielle Materialien Vanadiumoxid und mindestens ein Oxid eines Metalls, ausgewählt aus der Gruppe, bestehend aus: Niob, Magnesium, Molybdän und Seltenerdmetallen, umfaßt.

**7.** Verfahren nach Anspruch 5, wobei das Trägermaterial des oxidativen Dehydrierungs-Katalysators Siliziumoxid umfaßt.

**8.** Verfahren nach Anspruch 1, wobei der nachgeschaltete Katalysator einen endothermen Dehydrierungs-Katalysator umfaßt.

**9.** Verfahren nach Anspruch 8, wobei der endotherme Dehydrierungs-Katalysator mindestens eine Katalysatorzusammensetzung umfaßt, ausgewählt aus der Gruppe, bestehend aus:

A) einem Katalysator, umfassend Chromoxid und gegebenenfalls Oxide mindestens eines Metalls, ausgewählt aus der Gruppe, bestehend aus Mo, W, V, Ga, Mg, Ni, Fe, Alkalielementen, Erdalkalielementen und Seltenerdelementen,
B) einem Katalysator umfassend Vanadiumoxid und gegebenenfalls mindestens ein Element, ausgewählt aus der Gruppe, bestehend aus Li, Na, K und Mg,
C) einem Katalysator, umfassend Platin und gegebenenfalls mindestens ein Metall, ausgewählt aus der Gruppe, bestehend aus Natrium, Kalium, Cäsium, Rhenium und Zinn, und
D) einem Katalysator, umfassend mindestens ein Metall, ausgewählt aus der Gruppe, bestehend aus Ga, Fe, Mn und Co.

**10.** Verfahren nach Anspruch 8, wobei der endotherme Dehydrierungs-Katalysator ein Trägermaterial umfaßt.

**11.** Verfahren nach Anspruch 10, wobei das Trägermaterial des endothermen Dehydrierungs-Katalysators ein Material umfaßt, ausgewählt aus der Gruppe, bestehend aus: Aluminiumoxid, Titan, Zirkon, Siliziumoxid, Zeolithen, Seltenerdmetall-Oxid, gemischten Metalloxiden, mesoporösen Materialien, feuerfesten Materialien und Kombinationen davon.

**12.** Verfahren nach Anspruch 11, wobei der endotherme Dehydrierungs-Katalysator als essentielle Materialien Vanadiumoxid, Chromoxid und mindestens ein Metall, ausgewählt aus der Gruppe, bestehend aus: Kupfer, Silber und Gold, umfaßt.

**13.** Verfahren nach Anspruch 11, wobei das Trägermaterial des endothermen Dehydrierungs-Katalysators Siliziumoxid umfaßt.

**14.** Verfahren nach Anspruch 8, wobei das schwache Oxidationsmittel Kohlenstoffdioxid umfaßt.

**15.** Verfahren nach Anspruch 1, wobei ein Sauerstoff enthaltendes Gas getrennt von dem $C_2$-$C_4$ Alkan in dem exothermen Reaktionsbereich bereitgestellt wird.

**16.** Verfahren nach Anspruch 1, wobei mindestens ein Teil des schwachen Oxidationsmittels getrennt von dem $C_2$-$C_4$ Alkan in dem exothermen Reaktionsbereich bereitgestellt wird.

**17.** Verfahren nach Anspruch 1, wobei mindestens ein Teil des schwachen Oxidationsmittels getrennt von dem erwärmten, gemischten Produktgas in dem endothermen Reaktionsbereich bereitgestellt wird.

**18.** Verfahren nach Anspruch 1, wobei das $C_2$-$C_4$ Alkan Propan umfaßt, das entsprechende $C_2$-$C_4$ Alken Propen umfaßt und das schwache Oxidationsmittel Kohlenstoffdioxid umfaßt.

**Revendications**

**1.** Procédé pour la conversion d'un $C_2$-$C_4$ alcane en son $C_2$-$C_4$ alcène correspondant, ledit procédé comprenant les étapes de :

A) mise en contact d'un $C_2$-$C_4$ alcane et d'oxygène avec un catalyseur amont dans une zone de réaction exothermique, où le catalyseur amont est catalytiquement actif pour la conversion exothermique du $C_2$-$C_4$ alcane en son $C_2$-$C_4$ alcène correspondant, en présence d'oxygène ; et
B) conversion exothermique d'une partie du $C_2$-$C_4$ alcane en le $C_2$-$C_4$ alcène correspondant, dans la zone de réaction exothermique, pour produire un gaz de produits mélangés chauffé qui comprend le $C_2$-$C_4$ alcène correspondant, le $C_2$-$C_4$ alcane qui n'a pas réagi et la chaleur produite par ladite étape de conversion exothermique ;
C) mise en contact du gaz de produits mélangés chauffé et d'un oxydant faible avec un catalyseur aval dans une zone de réaction endothermique, où le catalyseur aval est catalytiquement actif pour la conversion endothermique du $C_2$-$C_4$ alcane qui n'a pas réagi en le $C_2$-$C_4$ alcène correspondant, en présence de l'oxydant faible ;
D) conversion endothermique d'au moins une partie du $C_2$-$C_4$ alcane qui n'a pas réagi en le $C_2$-$C_4$ alcène correspondant, dans la zone de réaction endothermique, pour produire un courant de produits cumulatifs qui comprend au moins le $C_2$-$C_4$ alcène correspondant produit dans chacune des zones de réaction.

**2.** Procédé selon la revendication 1 où le catalyseur amont comprend un catalyseur de déshydrogénation oxydative.

**3.** Procédé selon la revendication 2 où le catalyseur de déshydrogénation oxydative comprend au moins une composition de catalyseur choisie dans le groupe consistant en :

A) un catalyseur comprenant un ou plusieurs métaux nobles choisis parmi Pt, Pd, Rh, Ir et Ru ; et
B) un catalyseur comprenant au moins un oxyde d'un métal choisi parmi Li, Mo, W, V, Nb, Sb, Sn, Ga, Zr, Mg, Mn, Ni, Co, Ce et les métaux des terres rares.

**4.** Procédé selon la revendication 2 où le catalyseur de déshydrogénation oxydative comprend un matériau support.

**5.** Procédé selon la revendication 4 où le matériau support du catalyseur de déshydrogénation oxydative comprend un matériau choisi dans le groupe consistant en : l'alumine, le titane, le zirconium, la silice, les zéolites, les oxydes des métaux des terres rares, les oxydes métalliques mixtes, les matériaux mésoporeux, les matériaux réfractaires et leurs combinaisons.

**6.** Procédé selon la revendication 5 où le catalyseur de déshydrogénation oxydative comprend, comme matériaux essentiels, de l'oxyde de vanadium et au moins un oxyde d'un métal choisi dans le groupe consistant en : le niobium, le magnésium, le molybdène et les éléments des terres rares.

**7.** Procédé selon la revendication 5 où le matériau support du catalyseur de déshydrogénation oxydative comprend de l'oxyde de silicium.

**8.** Procédé selon la revendication 1 où le catalyseur aval comprend un catalyseur de déshydrogénation endothermique.

**9.** Procédé selon la revendication 8 où le catalyseur de déshydrogénation endothermique comprend au moins une composition de catalyseur choisie dans le groupe consistant en :

A) un catalyseur comprenant de l'oxyde de chrome et, éventuellement, des oxydes d'au moins un métal choisi dans le groupe consistant en Mo, W, V, Ga, Mg, Ni, Fe, les éléments alcalins, les éléments alcalino-terreux et les éléments des terres rares ;
B) un catalyseur comprenant de l'oxyde de vanadium et, éventuellement, au moins un élément choisi dans le groupe consistant en Li, Na, K et Mg ;

C) un catalyseur comprenant du platine et, éventuellement, au moins un métal choisi dans le groupe consistant en le sodium, le potassium, le césium, le rhénium et l'étain ;

et

D) un catalyseur comprenant au moins un métal choisi dans le groupe consistant en Ga, Fe, Mn et Co.

**10.** Procédé selon la revendication 8 où le catalyseur de déshydrogénation endothermique comprend un matériau support.

**11.** Procédé selon la revendication 10 où le matériau support du catalyseur de déshydrogénation endothermique comprend un matériau choisi dans le groupe consistant en : l'alumine, le titane, le zirconium, la silice, les zéolites, les oxydes des métaux des terres rares, les oxydes métalliques mixtes, les matériaux mésoporeux, les matériaux réfractaires et leurs combinaisons.

**12.** Procédé selon la revendication 11 où le catalyseur de déshydrogénation endothermique comprend, comme matériaux essentiels, de l'oxyde de vanadium, de l'oxyde de chrome et au moins un métal choisi dans le groupe consistant en : le cuivre, l'argent et l'or.

**13.** Procédé selon la revendication 11 où le matériau support du catalyseur de déshydrogénation endothermique comprend de l'oxyde de silicium.

**14.** Procédé selon la revendication 8 où l'oxydant faible comprend du dioxyde de carbone.

**15.** Procédé selon la revendication 1 où un gaz contenant de l'oxygène est fourni à la zone de réaction exothermique séparément du $C_2$-$C_4$ alcane.

**16.** Procédé selon la revendication 1 où au moins une partie de l'oxydant faible est fournie à la zone de réaction exothermique séparément du $C_2$-$C_4$ alcane.

**17.** Procédé selon la revendication 1 où au moins une partie de l'oxydant faible est fournie à la zone de réaction endothermique séparément du gaz de produits mélangés chauffé.

**18.** Procédé selon la revendication 1 où le $C_2$-$C_4$ alcane comprend du propane, le $C_2$-$C_4$ alcène correspondant comprend du propène et l'oxydant faible comprend du dioxyde de carbone.

EP 1 916 230 B1

## FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0117146 A
- US 5705684 A
- US 5086032 A
- US 20020077518 A
- US 20040034266 A
- US 6239325 B
- US 20050124840 A
- US 20050085678 A
- US 4046833 A
- US 4410752 A
- US 2500920 A
- US 5013706 A
- US 20040181107 A
- CN 1472005
- EP 1112241 A
- WO 2004054945 A

**Non-patent literature cited in the description**

- **ZHAORIGETU, B ; KIEFFER, R. ; HINDERMAN J.-P.** Oxidative Dehydrogenation of Propane on Rare Earth Vanadates. Influence of the Presence of CO2 in the feed. *Studies in Surface Science and Catalysis,* 1996, vol. 101, 1049-1058
- **LI, X.-H. ; LI, W.-Y ; XIE, K.-C.** Supported Vanadia Catalysts for Dehydrogenation of Ethylbenzene with CO2. *Catalyst Letters,* December 2005, vol. 105 (3-4
- **DURY, F ; GAIGNEAUX, E.M ; RUIZ, P.** The Active Role of CO2 at Low Temperature in Oxidation Processes: The Case of the Oxidative Dehydrogenation of Propane on NiMoO4 catalysts. *Applied Catalysis A: General,* 2003, vol. 242, 187-203
- **TAKEHIRA, K ; OISHI, Y ; SHISHIDO, T ; KAWABATA, T ; TAKAKI, K ; ZHANG, Q ; WANG, Y.** CO2 Dehydrogenation of Propane over Cr-MCM-41 Catalyst. *Studies in Surface Science and Catalysis,* 2004, vol. 153, 323-328